# EUROPEAN PATENT SPECIFICATION

(11) **EP 3 666 236 B1**
(45) Date of publication and mention of the grant of the patent: **05.05.2021**
(21) Application number: 18211968.5
(22) Date of filing: 12.12.2018
(51) Int. Cl.: A61F 9/007

(54) **GLAUCOMA IMPLANT DEVICE**
GLAUKOMIMPLANTATVORRICHTUNG
DISPOSITIF D'IMPLANT POUR GLAUCOME

(43) Date of publication of application: 17.06.2020
(73) Proprietor: AJL Ophthalmic, S.A., 01510 Miñano (Alava) (ES)
(72) Inventor: SPONSEL, William, Eric, San Antonio, Texas 78256 (US); LARRA MATEOS, Eva, 01510 MIÑANO (ÁLAVA) (ES); MARTINEZ EZKURRA, Sergio, 01510 MIÑANO (ÁLAVA) (ES); ÁLVAREZ PÉREZ, Iván, 01510 MIÑANO (ÁLAVA) (ES)
(74) Representative: Herrero & Asociados, S.L.

(56) References cited:
- US-A- 5 073 163
- US-A- 5 433 701
- US-A1- 2012 203 160
- US-A1- 2014 135 916
- US-A1- 2014 243 729
- US-B2- 9 561 133

## Description

### BACKGROUND OF THE INVENTION

### Field of the Invention

The present invention relates to devices for treating glaucoma by diverting aqueous humour out of the eye's anterior chamber through a surgically implanted duct passageway or shunt.

### Description of the Related Art

Increased pressure due to glaucoma can cause damage to the optic nerve, which can lead to blindness. Treatment strategies have focused on keeping the intraocular pressure down in order to preserve as much vision as possible and have included the use of drugs that lower intraocular pressure through various mechanisms. Unfortunately, drug treatments need much improvement, as they can cause adverse side effects and often fail to adequately control intraocular pressure. Moreover, lack of compliance leads to further symptom progression.

With respect to surgical procedures, one way to treat glaucoma is to implant a drainage device, or shunt, in the eye. The drainage device functions to drain aqueous humour from the anterior chamber and thereby reduce the intraocular pressure (IOP). The drainage device is typically implanted using an invasive surgical procedure. Pursuant to one such procedure, a flap is surgically formed in the sclera. The flap is folded back to form a small cavity and a shunt is inserted into the eye through the flap. Such a procedure can be quite traumatic as the implants are large and can result in various adverse events such as infections and scarring, leading to the need to re-operate. US 2013/0184631 discloses a surgical instrument in the form of a needle body for diverting aqueous humor from the operating site that tries to solve some of the drawbacks of existing devices. However, there is still a need for more reliable control of the intra ocular pressure.

Document US-A-2014/0243729 discloses a glaucoma implant according to the preamble of claim 1.

### SUMMARY OF THE INVENTION

The present invention provides a glaucoma implant device that provides better intraocular pressure control than prior devices. It consists of an elongate member with two tube-like sections of different diameter, the tube of larger diameter, or distal tube being fenestrated and provided with at least a fixation flange. The smaller diameter tube or proximal tube can be cut at an appropriate length during the operation, and is introduced directly into the anterior chamber and fixed with surgical tape. Diverting aqueous humor directly from the anterior chamber to the retrobulbar space obviates any need for on-globe blebs or shunts. The holes in the distal tube allow intercellular canaliculi to form between fat cells, maximizing the rate of aqueous humor resorption throughout the orbit.

### BRIEF DESCRIPTION OF THE DRAWINGS

To complete the description and provide for better understanding of the invention, a set of drawings is provided. Said drawings illustrate a preferred embodiment of the invention, which should not be interpreted as restricting the scope of the invention, but just as an example of how the invention can be carried out.

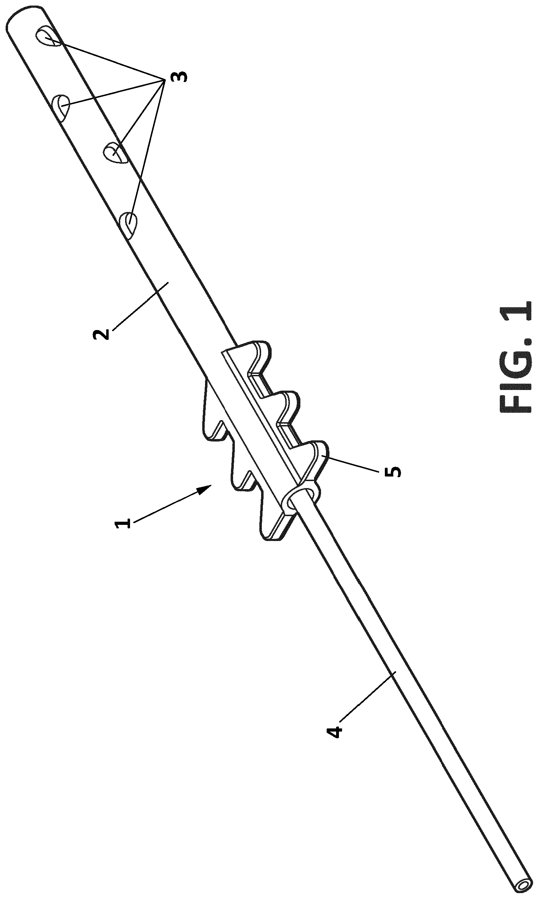

Figure 1 is a perspective view of the device of the invention.

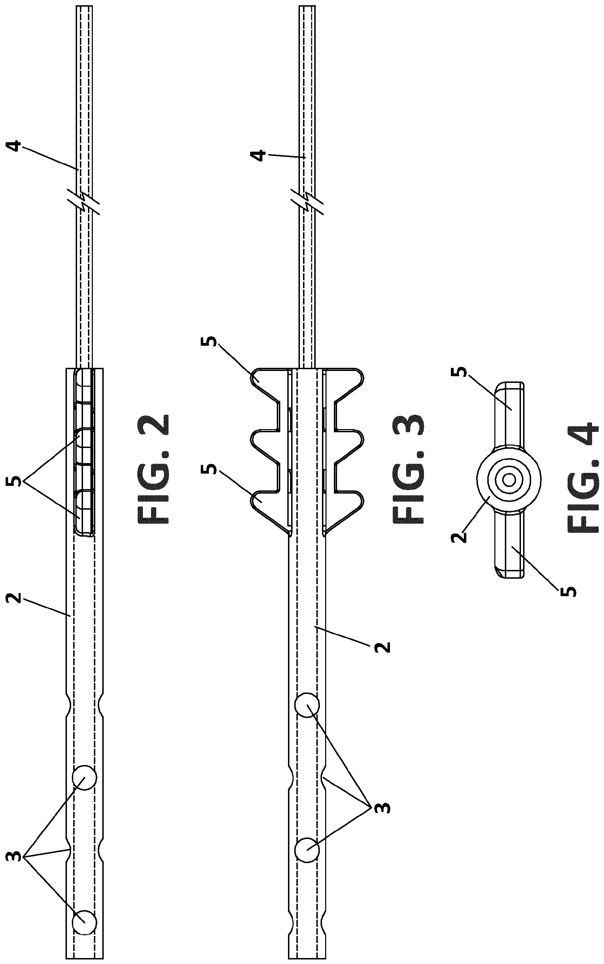

Figure 2 shows a lateral view.
In Figure 3, details of the fixation flanges are shown.
Figure 4 is a frontal view of the invention.

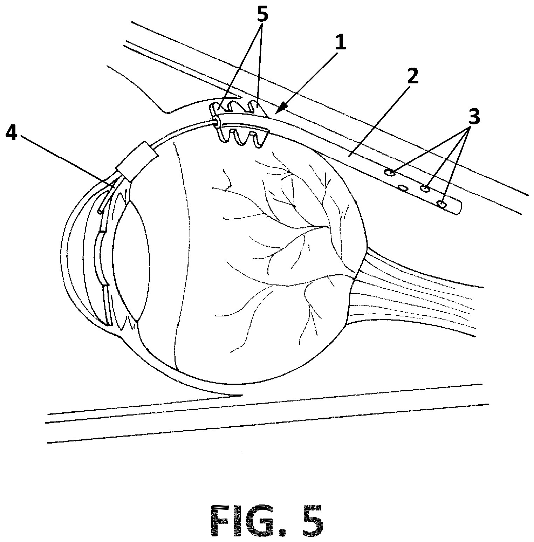

Figure 5 shows the implant once it is placed in the eye.
Figure 6 is a histogram showing experimental results demonstrating the effect of the invention.

### DESCRIPTION OF THE PREFERRED EMBODIMENTS

As used herein, the term "distal" is defined as in the direction of the eye of the patient and away from the surgeon that will implant the device. The term "proximal" is defined as in the direction of the surgeon.

The glaucoma implant device of the invention comprises an elongate member 1 made of surgical silicone or any of various other biocompatible polymers or gels. It is provided with two tube-like sections, both having an interior flow pathway. The flow pathway provides a fluid pathway between the eye's anterior chamber and a suprachoroidal space when the elongate member is implanted in the eye.

The proximal tube 4 is of the same diameter as that of a standard tube shunt, its lumen diameter lies between 0.1 and 0.25 mm and is longer than the distal tube 2. The distal tube 2, of a length between 10 to 20 mm, has a greater diameter, between 1 and 3 mm, is provided with holes (at least 2 and up to 30 in all directions) and presents one or more fixation flanges 5. The fixation flanges have a chevron or V shape with a capped vertex pointing towards the distal tube for facilitating insertion. Using a curved hollow trocar that fits snugly around the proximal tube 4, the device can be slid easily into the retrobulbar space via a small superior limbal dissection. The distal tube 2 can be then secured to the sclera anterior and to the upper fornix by passing prolene suture twice through the conjunctiva and around the flange or flanges 5, thereby minimizing any risk of anterior or posterior migration. The proximal tube 4 can then be cut to the appropriate length and introduced into the anterior chamber, with or without scleral reinforcement graft, using the standard shunt insertion method most familiar to the surgeon. The placement of this anterior-to-back device directly from the anterior chamber to the retrobulbar space has the advantage that a bleb can be dispensed with. The holes 3 produce much better and more reliable IOP control than devices with either no holes or holes that are too small. The optimal diameter for the holes 3 lies between 0.25 and 1.2 mm. Surgical experience confirms the unique importance of multiple omnidirectional holes. Devices with a non-fenestrated tube, too few holes, or holes of inadequate diameter do not attain satisfactory therapeutic levels of intraocular pressure reduction. This is evidently because, with more holes of adequate size, more networks of small intercellular canaliculi may form between fat cells, maximizing the rate of aqueous humor resorption throughout the orbit.

The distal tube 2 must be sufficiently rigid to have no risk of collapse or folding or buckling as it is passed into the retrobulbar space, but must be sufficiently malleable to avoid traumatizing the optic nerve or any of the other vascular or neural elements and extraocular muscles that occupy the retrobulbar space. At the same time, it must be sufficiently rigid to allow holes of the mentioned size to provide adequate aqueous regress between the bulging fat cells in each tube orifice to allow the formation of the interstitial canaliculi leading to the orbital periosteum in response the aqueous humor hydrostatic pressure gradient. A material with a tensile strength of 9 MPa to 11 MPa (90 bar to 110 bar) (around 10.3 MPa) is a suitable one. The tear strength lies around 9 to 11 MPa and its elongation is between 300 and 420 %.

The distal tube's lumen must be of sufficient diameter to allow fat cells to bulge into the holes thereby acting as a one-way non-return valve system for the flowing aqueous humor without allowing those bulging fat cells to significantly diminish flow through the full length of the lumen (or totally obstruct it by impinging the opposite wall of the tube). As the distal tube's diameter lies between 1 and 3 mm, the lumen must thus have a diameter of 0.8 - 1.2 mm.

### Experimental results:

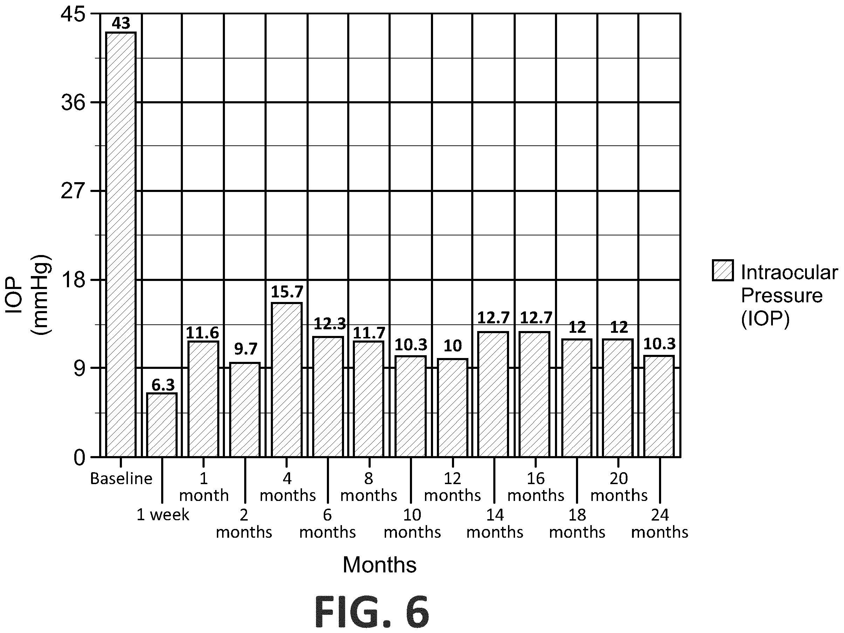

The implant was used in 9 patients having a mean preoperative IOP of 29.33 ± 3.07 mmHg, wherein 1 mmHg corresponds to 133.322 Pa, which was reduced to 14.28 ± 2.12 at week one post-implantation (mean % IOP Δ 51.5%), and to 20.12 ± 3.17, 17.75 ± 2.18, 18.33 ± 3.77, and 21 ± 2.94 at one, two, four, and six months post-implantation, respectively (mean % IOP Δ -31.5%, - 57%, -54%, -53%). In mmHg the implant of the invention thus reduced the mean IOP by 12.72 at one week, by 9.13 at one month, 12.00 at two months, 13.83 at four months, and 13.25 mmHg at six months. Figure 6 shows the results in a 24 month span for one of the patients.

In conclusion, the retrobulbar glaucoma treatment device of the invention offers the prospect of stable IOP control for eyes with severe and complex glaucoma aetiologies. The invention stabilizes progression of visual loss and reduces pharmacologic dependency, improving the quality of life for these patients. The ability to safely and easily divert aqueous humor directly from the anterior chamber to the retrobulbar space obviates any need for on-globe blebs or shunts. Retrobulbar shunts have thus far demonstrated safe and predictable results in the short-, intermediate- and long-term. This novel technology, has the potential to serve glaucoma patients throughout the world in for whom current established methods of treatment with medication, laser, MIGS, trabeculectomy, or standard primary tube shunts are either impracticable or fail to provide satisfactory outcomes. It is evident that the direct diversion of aqueous to the retrobulbar space as a primary procedure could provide a straightforward, rapid, limited-risk, and low-cost means to attain sustained and therapeutically desirable levels of IOP reduction.

As it is used herein, the term "comprises" and derivations thereof (such as "comprising", etc.) should not be understood in an excluding sense, that is, these terms should not be interpreted as excluding the possibility that what is described and defined may include further elements, steps, etc. On the other hand, the invention is obviously not limited to the specific embodiment(s) described herein, but also encompasses any variations that may be considered by any person skilled in the art (for example, as regards the choice of materials, dimensions, components, configuration, etc.) to be within the general scope of the invention as defined in the claims.

## Claims

1. Glaucoma implant device comprising an elongate member (1) made a biocompatible polymer or gel provided with two tube-like sections, a proximal tube (4) and a distal tube (2), both tubes having an interior flow pathway providing a fluid pathway between the eye's anterior chamber and a suprachoroidal space when the elongate member (1) is implanted in the eye, the proximal tube (4) having a lumen diameter between 0.1 mm and 0.25 mm, the distal tube (2) having a diameter between 1 and 3 mm and greater than that of the proximal tube (4), the distal tube being provided with at least two holes (3) in all directions **characterised in that** the distal tube is provided with at least one fixation flange (5), wherein the at least one fixation flange has a chevron or V shape with a capped vertex pointing towards the distal tube for facilitating insertion.

2. Glaucoma implant device according to claim 1 wherein the biocompatible polymer or gel has a tensile strength of 9 MPa to 11 MPa.

3. Glaucoma implant device of any of the previous claims wherein the holes (3) of the distal tube (2) have a diameter between 0.25 mm and 1.2 mm.

4. Glaucoma implant device according to any of the previous claims wherein the lumen of the distal tube (2) has a diameter of 0.8 mm to 1.2 mm.

## Patentansprüche

1. Glaukomimplantatvorrichtung, aufweisend ein längliches Element (1) aus biokompatiblem Polymer oder Gel, das mit zwei röhrenartigen Abschnitten, einer proximalen Röhre (4) und einer distalen Röhre (2), versehen ist, wobei beide Röhren einen inneren Strömungsweg aufweisen, der einen Fluidweg zwischen der vorderen Augenkammer und einem suprachoroidalen Raum bereitstellt, wenn das längliche Element (1) in das Auge implantiert ist, wobei die proximale Röhre (4) einen Lumendurchmesser zwischen 0,1 mm und 0, 25 mm aufweist, die distale Röhre (2) einen Durchmesser zwischen 1 und 3 mm und größer als den der proximalen Röhre (4) aufweist, wobei die distale Röhre mit mindestens zwei Löchern (3) in alle Richtungen versehen ist, **dadurch gekennzeichnet, dass** die distale Röhre mit mindestens einem Befestigungsflansch (5) versehen ist, wobei der mindestens eine Befestigungsflansch eine Zickzack- oder V-Form mit einem gekappten Scheitelpunkt aufweist, der in Richtung der distalen Röhre zeigt, um das Einführen zu erleichtern.

2. Glaukomimplantatvorrichtung nach Anspruch 1, wobei das biokompatible Polymer oder Gel eine Zugfestigkeit von 9 MPa bis 11 MPa aufweist.

3. Glaukomimplantatvorrichtung nach einem der vorhergehenden Ansprüche, wobei die Löcher (3) der distalen Röhre (2) einen Durchmesser zwischen 0,25 mm und 1,2 mm aufweisen.

4. Glaukomimplantatvorrichtung nach einem der vorhergehenden Ansprüche, wobei das Lumen der distalen Röhre (2) einen Durchmesser von 0,8 mm bis 1,2 mm aufweist.

## Revendications

1. Dispositif implantaire pour glaucome comprenant un élément allongé (1) fait d'un polymère biocompatible ou d'un gel pourvu de deux sections en forme de tube, un tube proximal (4) et un tube distal (2), les deux tubes présentant une voie d'écoulement intérieur fournissant un passage de fluide entre la chambre antérieure de l'œil et un espace suprachoroïdien lorsque l'élément allongé (1) est implanté dans l'œil, le tube proximal (4) présentant un diamètre de lumière compris entre 0,1 mm et 0,25 mm, le tube distal (2) présentant un diamètre compris entre 1 et 3 mm et plus que celui du tube proximal (4), le tube distal étant pourvu d'au moins deux trous (3) dans toutes les directions **caractérisé en ce que** le tube distal est pourvu d'au moins une bride de fixation (5), dans lequel la au moins une bride de fixation (5) présente une forme de chevron ou de V avec un sommet coiffé pointant vers le tube distal pour faciliter l'insertion.

2. Dispositif implantaire pour glaucome selon la revendication 1, dans lequel le polymère ou gel biocompatible présente une résistance à la traction de 9 MPa bars à 11 MPa.

3. Dispositif implantaire pour glaucome selon l'une quelconque des revendications précédentes, dans lequel les trous (3) du tube distal (2) présentent un diamètre compris entre 0,25 mm et 1,2 mm.

4. Dispositif implantaire pour glaucome selon l'une quelconque des revendications précédentes, dans lequel la lumière du tube distal (2) présente un diamètre de 0,8 mm à 1,2 mm.
